Europäisches Patentamt

European Patent Office

Office européen des brevets

(11) Publication number: **0 413 027 A1**

# EUROPEAN PATENT APPLICATION
## published in accordance with Art. 158(3) EPC

(21) Application number: 89908507.0

(22) Date of filing: 21.07.89

(86) International application number:
PCT/JP89/00734

(87) International publication number:
WO 90/01538 (22.02.90 90/05)

(51) Int. Cl.5: **C12M 1/40**, //C02F3/10

(30) Priority: 29.07.88 JP 190322/88

(43) Date of publication of application:
20.02.91 Bulletin 91/08

(84) Designated Contracting States:
DE FR GB

(71) Applicant: **KIRIN BEER KABUSHIKI KAISHA**
**26-1, Jingumae 6-chome**
**Shibuya-ku Tokyo 150(JP)**

Applicant: **Bridgestone Corporation**
**10-1, Kyobashi 1-Chome Chuo-Ku**
**Tokyo 104(JP)**

(72) Inventor: **SUZUKI, Akira Kirin Beer Kabushiki**
**Kaisha**
26-1, Jingumae 6-chome
Shibuya-ku Tokyo 150(JP)
Inventor: **MATSUDA, Shoichi Kirin Beer**
**Kabushiki Kaisha**
26-1, Jingumae 6-chome Shibuya-ku
Tokyo 150(JP)
Inventor: **NARUMIYA, Tsuneaki**
6-8, Hosen 2-chome Hodogaya-ku
Yokohama-shi Kanagawa 240(JP)
Inventor: **ODAKA, Fumio BS Apartment 3-B-1**
827, Kashio-cho Totsuka-ku
Yokohama-shi Kanagawa 244(JP)

(74) Representative: **Reichel, Wolfgang, Dipl.-Ing.**
**et al**
**Reichel und Reichel Parkstrasse 13**
**D-6000 Frankfurt am Main 1(DE)**

(54) **BIOREACTOR USING OPEN-CELL POROUS CERAMIC CARRIER.**

(57) The title bioreactor contains therein an open-cell porous ceramic carrier which comprises a pore-forming, ceramic skeleton of a three-dimensional network structure having interconnected internal spaces formed by a number of fine pores with the skeleton being formed by a continuous saddle surface.

FIG. 2

# A BIOREACTOR USING A CONTINUOUSLY POROUS CERAMIC MATRIX

## TECHNICAL FIELD

This invention relates to a bioreactor, especially a bioreactor having an improved matrix used therein.

## BACKGROUND ART

It is conventional that a bioreactor as a reactor for obtaining a required product using as a catalyst, cells, enzymes, other intracellular substances, fungi or others (hereinafter generically called biocatalyst) contains a carrier for a biocatalyst to adhere to.

Conventionally known carriers or matrices which are used in such bioreactor are i) entrapping method matrices, such as calcium alginate, carageenan gels, etc., ii) porous bead carriers, as of ceramics, plastics, metal, etc., iii) honeycomb-shaped through-pore matrices, as of sintered ceramics and plastics.

But these conventional carriers or matrices have the following problems in use especially in aerobic reactors or those involving gas generation.

That is, with i) entrapping method matrices, which are organic substances, the problems are unreliable stability in long running operations and deterioration of the matrices due to swelling. Another problem with these matrices results from that these matrices are generally globuler per se and occupy $\pi/6$ of a volume of a reactor. Consequently the space utilization efficiency of a reactor becomes lower; these matrices are laid on one another in a reactor, and contacts among these matrices define restricted parts where gas becomes stagnant, and biocatalysts tend to clog. A further problem is that the spherical interiors of the matrices per se tend to grow anaerobic conditions.

A problem with ii) porous bead carrier is that, similarly with i) entrapping method matrices, the bead carriers are laid on one another to adversely define a number of restricted parts in a reactor.

An additional problem is that gas and liquid stagnate in air cavities in porous beads to tend to create anaerobic conditions, and because of the gas trapped in the air cavities the porous beads decrease their specific gravities and tend to float.

Problems with iii) through-pore matrices, which have straight through-pores formed in the matrices, are that, in the case where a sprinkler system is used, liquid does not reside long nor frequently collide with biocatalysts, and the liquid falls so quickly, the gas rises so quickly that the liquid is in contact with biocatalysts for very short periods of time. Another problem with through-pore matrices is that as in the case of cross flow matrices, biocatalysts tend to peel off. An additional problem is that the through-pore matrices per se are expensive.

An object of this invention is to provide a bioreactor which improves the above-described problems of the respective conventional matrices. That is, an object of this invention is to provide a bioreactor using a continuously porous ceramic matrix which has a high porosity and a high space utilization factor because of no restricted parts and acute-angled parts, is free from liquid and gas stagnation, which increases the frequency of the gas-liquid contact, and which has a large area for biocatalysts to adhere to, and improves their adhesion.

## DISCLOSURE OF THE INVENTION

This invention relates to a bioreactor equipped with a continuously porous ceramic matrix interior to the vessel which comprises pore-defining frames each having a three-dimensional network structure with communicated interior spaces defining a number of pores in continuous communication with one another, and each pore-defining frame including continuously repeated saddle-shaped surfaces.

## BRIEF DESCRIPTION OF THE DRAWINGS

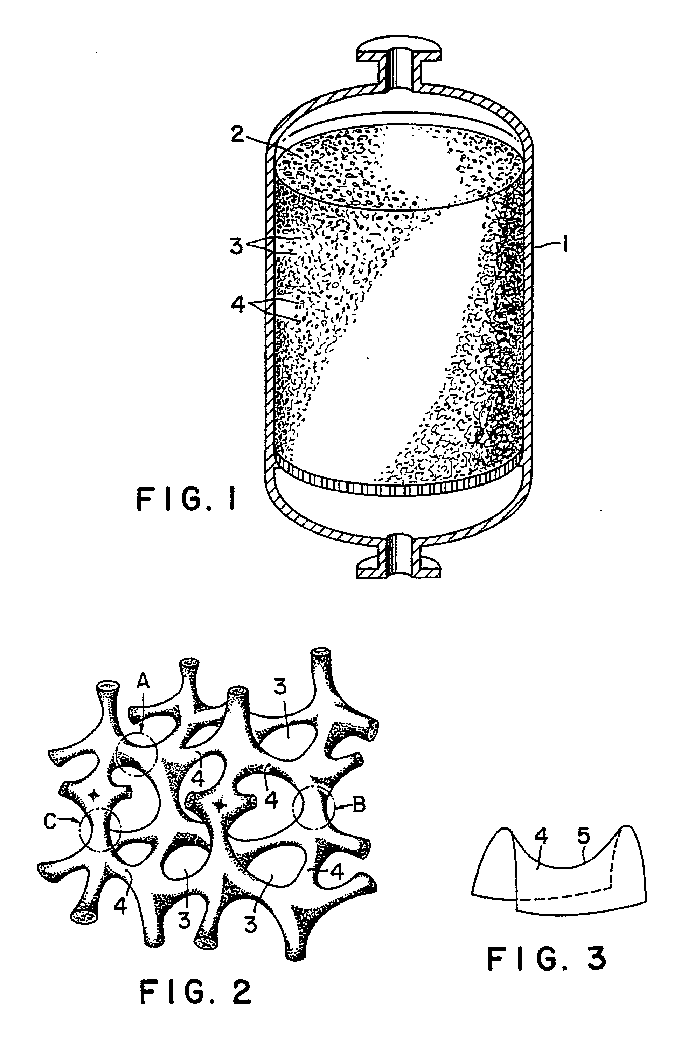

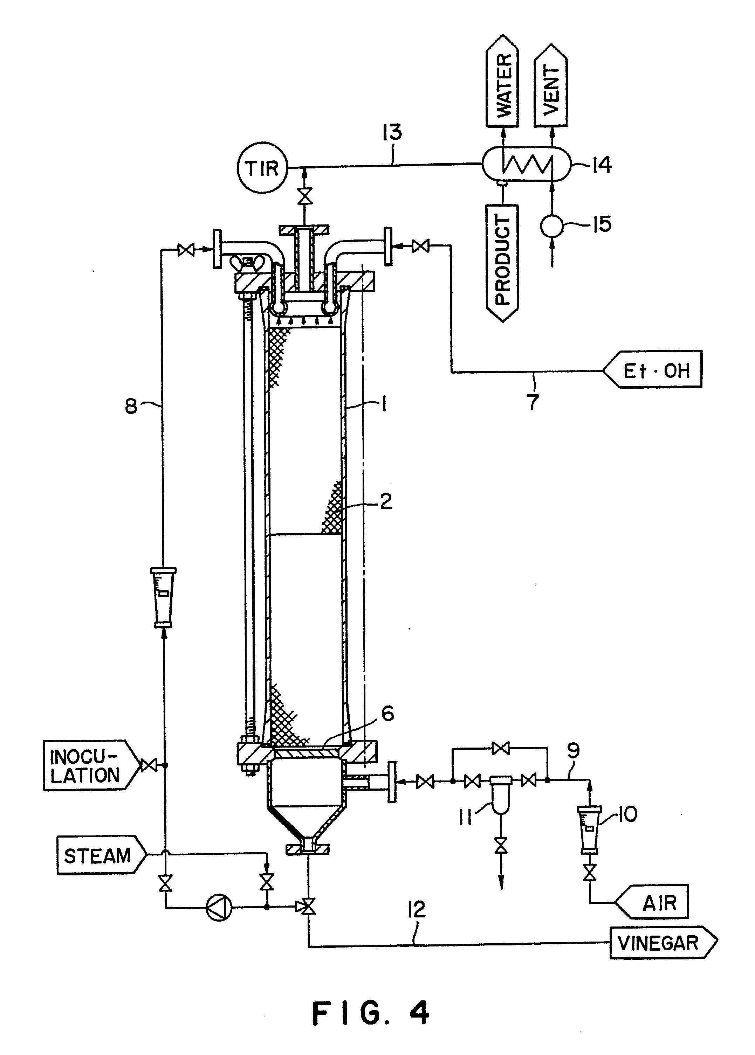

FIG. 1 is a vertical sectional view of a major part of this invention; FIG. 2 is an enlarged perspective view of a part of the continuously porous ceramic matrix; FIG. 3 is a perspective view of portions (A), (B) and (C) in FIG. 2; and FIG. 4 is a schematic view of an example of the application of this invention to vinegar production.

## BEST MODE FOR CARRYING OUT THE INVENTION

In this invention, as generally shown in FIG. 1 and partially enlarged in FIG. 2, a continuously porous ceramic matrix 2 to be loaded in a reactor vessel 1 is made of ceramic and is formed in a three-dimensional network structure having communicated interior spaces defining a number of pores 3 in continuous communication with one another. Each of the pore-defining frames 4 defining pores 3 has saddle-shaped surfaces 5 (curved surface similar to hyperbolic paraboloid).

The ceramics is exemplified by cordierite

$(2MgO \bullet 2Al_2O_3 \bullet 5SiO_2)$ + alumina $(Al_2O_3)$ , etc. The pores 3 are sized so as to be present by around 2 ~ 40 per 25 mm, and pores having a porosity of 70 ~ 90% are selected for use.

The continuously porous ceramic matrix 2 is formed by adhering a slurry of ceramic fine grains as a raw material to, e.g., a skeleton of polyurethane foam without the cell membranes, drying the slurry to cure the ceramics, and sintering the ceramics at a high temperature into a ceramic body while carbonizing to remove the skeleton of polyurethane foam. A thus prepared body is formed into a size contoured to an interior shape and a volume of the reactor vessel 1, and is loaded in the reactor vessel 1.

FIG. 4 shows an example of the application of the reactor using the continuously porous ceramic matrix 2 to vinegar production. A perforated support plate 6 is fixedly supported in the reactor vessel 1 at a lower position. On the upper side of the support plate 6 is supported a cylindrical and continuously porous ceramic matrix 2 which substantially agrees with the inner diameter of the reactor vessel 1.

An ethanol supply unit 7 and a fungus supply unit 8 are connected to the upper portion of the reactor vessel 1 in communication with the interior of the reactor vessel 1. A compressed air supply unit 9 for supplying compressed air from a compressor is connected to the portion of the reactor vessel 1 below the support plate 1 through a flow meter 10 and a filter 11. A recovery unit 13 is connected to the top of the reactor vessel 1 through a cooler 14 and a pump 15.

This reactor is operated in accordance with the usual vinegar producing process. A biomass supplied into the reactor vessel 1 moves down through the pores 3 of the continuously porous ceramic matrix during which the biomass is adhered to the surfaces of the pore-defining frames 4. On the other hand, ethanol flows downward in an undirected manner through the pores 3 during which contact between the ethanol and the biomass takes place frequently with the result of a preferable reaction. Since the continuously porous ceramic matrix 2 is free from restricted portions and gas cavities, no liquid and gas stagnation takes place during the above-described flow, and accordingly even aerobic reactors properly function with the continuously porous ceramic matrix 2.

In addition, in culturing adhesive animal cells, etc., with the presently known techniques it is difficult to separate a cellular product (object substance) from the components of serum. Because of this difficulty, in some cases they are cultured at the beginning on a medium using serum for their adhesion and rinsed with phosphoric acid buffer or others to be subsequently cultured on a serum-free medium. In the conventional porous carriers, such as microcarriers, porous glass beads, etc., it is impossible in this rinse operation to perfectly rinse the interiors of air cavities with rinse liquid several times to tens of times an amount of a medium. But reactors using the continuously porous ceramic matrix according to this invention permit the rinse operation to be perfectly conducted with rinse liquid 1/5 to one times an amount of a medium.

As described above, according to this invention, the matrix to be loaded in a reactor vessel has a structure which pore-defining frames of ceramics define a number of pores which are arranged at random and are in communication with one another. Accordingly the space utilization factor of the reactor is so high that no stagnation takes place even though liquid and gas flow along long paths. The reactor is suitable to be used especially as an aerobic reactors. Each pore-defining frame has continuously repeated saddle-shaped surfaces. The area for a biocatalyst to adhere to is accordingly increased, and its adhesion is enhanced, which, coupled with the longer paths described above, contributes to more frequent contact between the biocatalyst, and the gas and liquid, with the results of a much improved reaction efficiency and no clogging. Furthermore, in a rinse operation, rinse water passes through all the pores and consequently, the matrix can be perfectly rinsed with a small amount of water. In addition, this matrix can be less expensive compared with the through-pore matrices. Thus, the prices of reactors can be reduced, and reactors of high quality can be provided.

It is possible that at least the surface of the pore-defining frame is made of an electrically active substance. Specifically, the whole frame 4 is made of a solid electrolyte, or the surface of the frame 4 is coated with an electrically conductive substance. This solid electrolyte has a high electric load in a medium and, in many cases, has a negative charge.

As the solid electrolyte, $\beta$-alumina, $\beta$-alumina-lithia, $\beta$-alumina-magnesia, partially stabilized zirconia, etc. are selected. As the electrically conductive substance are selected carbon-content latex, and silicides, borides and carbides of d-type metals, such as titanium boride, molybdenum bisilicide, tungsten carbide, etc., which have an electrical conductivity equal to or higher than $10^3$ mho/cm, and preferably equal to or higher than $10^4$ mho/cm. Its coating amount is preferably 0.1 ~ 20 weight %.

The ceramics as a base material of the pore-defining frame 4 used in the case where the electrically conductive substance is coated on the frame 4 is cordierite + alumina or others.

By making the whole or the surfaces of the pore-defining frames constituting the matrix of the electrically active substance, e.g., the electrolyte or electrically conductive substance, as described above according to this invention, a biocatalyst can be securely fixed to the frames by its self-adhesion due to its intrinsic charge (ions) when the matrix is loaded in a reactor. Consequently, the adhesion of the biocatalyst is enhanced, and as a result, the adhesion factor can be drastically enhanced.

Each frame 4 of the matrix may have a concave and convex portion formed in the surface thereof. The concavities of this concave and convex portion has a size of 10 ~ 300 $\mu$ in diameter and 1 ~ 100 $\mu$ in depth corresponding to that of a biocatalyst to be used, i.e., 5 ~ 15 $\mu$ for yeast, 50 ~ 100 $\mu$ for animal cells and 30 ~ 40 $\mu$ for moulds.

The concave and convex portion is formed by adhering a different substance, e.g., inflammable powder, to the surface of frame 4 when the matrix 2 is formed before the surface of the frame with the above-described slurry of fine grains of ceramics as a raw material adhered to, and drying and sintering the whole. Otherwise, the concave and convex portion can be formed by coating the surface of the frame with active alumina to thereby rough the surface. Concavities may be formed in the above-described size. The active alumina is used by 5 ~ 50 weight % of the frames 4.

By forming the concave and convex portion in the surfaces of the frames defining the pores of the matrix as described above according to this invention, the area for a biocatalyst to adhere to is increased, and the biocatalyst is caught in the concavities to settle therein without being peeled off by flows of liquid, when the matrix is loaded in a reactor. Thus, the adhesion of a biocatalyst to the surface of the matrix and the adhesion factor are drastically enhanced. The matrix can extremely enhance the performance of bioreactors including chemical reactors.

The matrix 2 may be honeycomb-shaped and have honeycomb-shaped pores, or may be lattice-shaped and have rectangular pores. Thus-shaped matrices can also drastically improve the adhesion factor of a biocatalyst compared with conventional similarly shaped matrices.

## INDUSTRIAL APPLICABILITY

As described above, a bioreactor using the continuously porous ceramic matrix according to this invention can be used widely as a bioreactor covering a chemical reactor for vinegar production or other purposes, and which has much improved adhesion of a biocatalyst to the matrix and the immobilization.

## Claims

1. A bioreactor using a continuously porous ceramic matrix comprising; pore-defining frames of ceramics each having a three-dimensional network structure with communicated interior spaces defining a number of pores in continuous communication with one another, said pore-defining frames forming said continuously porous ceramic matrix by the continuously repeating saddle-shaped surfaces thereof, and said matrix being equipped in a reactor vessel of said bioreactor.

2. The bioreactor using a continuously porous ceramic matrix according to claim 1, wherein the porosity of the continuously porous ceramic matrix is 70 ~ 90%, and the number of the pores is 2 ~ 40 per 25 mm.

3. The bioreactor using a continuously porous ceramic matrix according to claim 1, wherein said pore-defining frames have at least the surfaces formed of an electrically active substance.

4. The bioreactor using a continuously porous ceramic matrix according to claim 3, wherein said electrically active substance is a solid electrolyte.

5. The bioreactor using a continuously porous ceramic matrix according to claim 1 or 2, wherein said pore-defining frames have the surfaces coated with an electrically active substance.

6. The bioreactor using a continuously porous ceramic matrix according to claim 5, wherein said electrically active substance is an electrically conductive substance.

7. The bioreactor using a continuously porous ceramic matrix according to claim 1, wherein said pore-defining frames have concave and convex portions formed in said pore-defining portions.

8. The bioreactor using a continuously porous ceramic matrix, wherein the cavities of the concave and convex portions have a diameter of 10 ~ 300 $\mu$ and a depth of 1 ~ 100 $\mu$.

9. The bioreactor using a continuously porous ceramic matrix according to claim 1, wherein said pore-defining frames have the surfaces coated with active alumina to thereby form pores of below sub-micron.

10. The bioreactor using a continuously porous ceramic matrix according to claim 5, wherein said pore-defining frames have their surfaces coated with active alumina and formed in concavities and convexities.

11. The bioreactor using a continuously porous ceramic matrix according to claim 9, wherein said active alumina is 5 ~ 50 weight % of said pore-defining frames.

12. The bioreactor using a continuously porous ceramic matrix according to claim 1, wherein said pores are defined in the honeycomb shape by said pore-defining frames.

13. The bioreactor using a continuously porous ceramic matrix according to claim 1, wherein said pores are defined in a rectangular shape by said pore-defining frames.

FIG. 1

FIG. 2

FIG. 3

F I G. 4

# INTERNATIONAL SEARCH REPORT

International Application No PCT/JP89/00734

## I. CLASSIFICATION OF SUBJECT MATTER (if several classification symbols apply, indicate all) [6]

According to International Patent Classification (IPC) or to both National Classification and IPC

Int. Cl$^4$    C12M1/40//C02F3/10

## II. FIELDS SEARCHED

| Minimum Documentation Searched [7] | |
|---|---|
| Classification System | Classification Symbols |
| IPC | C12M1/00, 1/40, C02F3/10 |

| Documentation Searched other than Minimum Documentation to the Extent that such Documents are Included in the Fields Searched [8] |
|---|
| Jitsuyo Shinan Koho         1926 - 1988 |
| Kokai Jitsuyo Shinan Koho   1971 - 1988 |

## III. DOCUMENTS CONSIDERED TO BE RELEVANT [9]

| Category [*] | Citation of Document, [11] with indication, where appropriate, of the relevant passages [12] | Relevant to Claim No. [13] |
|---|---|---|
| X | JP, A, 63-62594 (Nippon Steel Corporation) 18 March 1988 (18. 03. 88) & DE, A1, 3726201 & US, A, 4832847 | 1 - 2, 7 - 13 |
| A | JP, A, 63-177792 (Nippon Sharyo Seizo Kaisha, Ltd.) 21 July 1988 (21. 07. 88) (Family: none) | 1 - 2, 7 - 13 |
| Y | JP, A, 63-62594 (Nippon Steel Corporation) 18 March 1988 (18. 03. 88) & DE, A1, 3726201 & US, A, 4832847 | 3 - 6 |
| Y | JP, A, 63-31595 (Kensetsu-sho Doboku Kenkyusho-cho) 10 February 1988 (10. 02. 88) (Family: none) | 3 - 6 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
|---|---|
| October 9, 1989 (09. 10. 89) | October 23, 1989 (23. 10. 89) |
| International Searching Authority | Signature of Authorized Officer |
| Japanese Patent Office | |

Form PCT/ISA/210 (second sheet) (January 1985)